# EUROPEAN PATENT APPLICATION

(11) **EP 3 267 175 A1**
(43) Date of publication of application: **10.01.2018**
(21) Application number: 16760946.0
(22) Date of filing: 03.03.2016
(51) Int. Cl.: G01N 1/28, G01N 23/083, A61B 6/12

(54) **AUXILIARY DEVICE FOR LOCATING, MAPPING AND MICROSCOPICALLY MEASURING NEOPLASIAS**

(30) Priority: 06.03.2015 BR 10154994
(71) Applicant: Fundação Antônio Prudente, 01509-900 São Paulo - SP (BR); Moreira Pedao, Diego, 04691-090 São Paulo (BR)
(72) Inventor: MOREIRA PEDAO, Diego, 04691-090 São Paulo (BR); PIANA DE ANDRADE, Victor, 04120-080 São Paulo (BR)
(74) Representative: Michalski Hüttermann & Partner Patentanwälte mbB
(86) International application number: PCT/BR2016/050047
(87) International publication number: WO 2016/141444

(57) **Abstract**

The present invention refers to an auxiliary device for locating, mapping and microscopically measuring neoplasias, in particular microcalcifications in breast surgery specimens, linked to a radiological procedure.

## Description

### FIELD OF THE INVENTION

The present invention refers to an auxiliary device for locating, mapping and microscopically measuring neoplasias, in particular microcalcifications in breast surgery specimens, linked to a radiological procedure.

The device of the invention exerts a number of valuable functions, allowing a surgical specimen, the result of an excision:
(1) during the intraoperative period to be properly fixed to undergo radiography, allowing precise location of microcalcifications and decision on the need for surgical expansion,
(2) to be efficiently conserved by immersion in formaldehyde, in the same position and shape it was radiographed;
(3) to provide the mapping of the microcalcifications and microscopic measurement of tumor size for accurate pathological staging, depending on the radiographic quality and print of the matrix left in the specimen.

### Background of the invention

Breast cancer accounts for 10% of all cancers diagnosed worldwide and is the most common cancer in women, without about 25% of tumors of this kind.

The use of mammography as a population screen method has permitted a large decrease in the mortality rates of the disease by anticipating diagnosis, simplifying treatment and its side effects. Patients diagnosed early and restricted to breast tumors are in the good prognosis group, and can be spared from more aggressive treatments. About 25% of cancer cases is diagnosed in its *in situ* stage, that is, when it has not invaded the breast stroma and is considered technically curable. The mammographic screen gains are most evident in the population aged 50-69 years where 6 to 9 deaths from breast cancer are prevented every 1000 women who undergo the examination annually.

Today breast cancer has five treatment options, namely: surgery, radiotherapy, hormonioterpia, terapia anti-HER2 and chemotherapy. Each patient receives a particular combination of these options depending on their tumor characteristics. Tumor size is one of the most important variables in this decision. Small tumors, called Stage 1 (up to 2 cm) can be divided into T1a (up to 5mm), T1b (between 5 and 10mm) and T1c (between 10 and 20mm). These categories define the indication of chemotherapy.

One of the cancer-suspicious findings most often detected by mammography are clustered microcalcifications without associated tumor mass. These microcalcifications may represent invasive carcinoma areas (generally at an early stage), carcinoma *in situ* or both and only microscopic examination can make a definitive diagnosis and tumor measurement. Microcalcifications not associated with palpable masses represent a group of breast lesions that require a unique sequence of pre- and post-operative to ensure the quality of the diagnosis:
1 - Pre-operative location of microcalcifications by needling or radiolabelling;
2 - Radiography of the surgical specimen during the intraoperative period to ensure that the entire area of interest has been excised surgically; this step is the object of major attention, since a fast and efficient surgical decision depends on its quality;
3 - Immediate fixation of the surgical specimen in a suitable volume of formaldehyde to ensure morphological quality to microscopy and preservation of the DNA and proteins for predictive and prognostic molecular tests;
4 - Histological mapping of the surgical specimen to allow the correct evaluation of the size of tumors.

The state of the art bears devices that are suited to just part of these needs, such as international application WO2014039727, but which are not suitable, for example, to contact with formaldehyde and to microscopic measurement.

From the above requirements, an auxiliary device was created for locating, mapping and microscopically measuring tumors, simple and efficient to work, providing advantages in relation to others existing in the state of the art.

### DESCRIPTION OF THE INVENTION

The description of the invention below is to facilitate the understanding, and not to impose any limits thereon other than those expressed in the accompanying claims.

The invention refers to an auxiliary device for locating, mapping and measuring neoplasias comprising an upper plate and a lower plate, made of atoxic material, wholly or substantially radio-transparent, between which a surgical specimen is placed, for example a breast fragment resulting from surgical excision. The degree of radiotransparency should allow visualization of the microcalcifications and the mapping matrix simultaneously.

Said upper plate comprises two cooperating parts, namely a frame and a movable center cover (for example tilting or sliding in relation to the frame, with means or devices having movement mechanically necessary for such purpose), and preferably the frame completely surrounds the central cover. At least the tilting cover is made of material wholly or substantially radio-transparent, for example methyl methacrylate-type plastic. The cover is movable, and may remain closed (thus endowed with a suitable measure of closure), or be opened exposing the central region of the frame, which gives access to a matrix of threads, preferably having low radio-opacity, flexible and high chemical resistance to products such as formaldehyde, for example nylon. The matrix of threads forms regular sized and spaced squares. Said matrix of threads is physically located beneath the cover of the upper plate, substantially parallel thereto, so that it can make physical contact with the surgical specimen that is contained between the upper and lower plates.

Said lower plate is preferably solid, composed, at least in its central region, of material that is also wholly or substantially radio-transparent and resistant to chemicals like formaldehyde, for example methyl methacrylate-type plastic, which is preferably visually transparent.

Said upper and lower plates are substantially parallel, after being moved closer to each other, to fix together the surgical specimen, such as by means of nuts associated to bolts whose longitudinal rods pass through both plates in substantially aligned openings, for example, at the edges of the frame of the upper plate and on the edges of the lower plate. Any other equivalent approximating means designed to bring together and fix the two plates together is suitable, as known in the art, designed to immobilize the surgical specimen adequately, for example, tightening clamps, sergeant clamps, ratchet with straps, etc., resistant to chemicals such as formaldehyde.

The approximation between the upper and lower plates, as well as the flexibility of the threads of the matrix of threads act to immobilize the surgical specimen between said plates. Although one comes close to the other, the upper and lower plates do not meet, by virtue of the volume of the immobilized surgical specimen between them.

The device of the invention can be endowed with means for slanting same, for example, to obtain the positioning of the device at 45 degrees, so as to modify the position of the surgical specimen during the radiographic procedure.

The following description refers to the form of using the device of the invention, as well as the advantages offered by using same.

Once the surgical specimen is withdrawn from the patient undergoing partial or total excision of a breast, the specimen is placed on the lower plate of the device, in a location substantially centralized in relation to the cover of the upper plate, and the upper plate is placed thereon, with its cover in closed position.

The upper and lower plates are then pressed together by means of approximation, such as progressive threading of the nuts onto the bolts, so as to immobilize the surgical specimen firmly, without too much compression that might destroy or substantially change its physical structure, after discrete elastic accommodation of the matrix of threads.

Therefore, as soon as possible, the surgical specimen, fixed between the upper and lower plates of the device of the invention, is subjected to a radiographic procedure, in equipment suited for such purpose, especially for detecting microcalcifications without palpable masses. In the radiographic record, the matrix of threads enables the recording of the location of the microcalcifications. Preferably, but without excluding any other alternative, the threads are made of low radio-opacity material, so that even the smallest microcalcifications located under the junction of two threads can be detected by the radiological procedure.

Also preferably, there are visible markings placed on the frame of the upper plate, for example with letters and numbers, enabling the safe designation of squares where microcalcifications are located during mapping.

After the radiography, the cover of the upper plate is opened, and the device is entirely immersed in formaldehyde. Different to other devices from prior art, the opening of the cover of the upper plate enables greater contact of the surgical specimen with the formaldehyde, compared with an upper plate without opening, allowing suitable conservation for the steps of histological processing, histological cut, microscopic interpretation and application of complementary molecular tests.

An additional advantage of setting the specimen in contact with the matrix of threads causes a physical impression thereof on the surface of the specimen, also allowing the squares of interest to be marked with various paint colors, such that when the specimen is removed from the device the topographic information is not lost.

In particular, the upper and lower plates of the device of the invention have the same width and length, in any desired size.

Preferably, all the components of the device of the invention have sizes suited to the type of radiological equipment to which they will be exposed, as well as the mechanical forces to which they are submitted, maintaining the best radiotransparency, where necessary, and chemical resistance to contact with formaldehyde.

Preferably, all the components of the device of the invention are washable and sterilizable in accordance with regular hospital and laboratory procedures for such so that it may be reused.

In another aspect of the invention is the use of the device described, characterized by comprising the following steps:
(a) placing a surgical specimen between said upper and lower plates;
(b) bringing said plates close to each other, pressing the surgical specimen between them, with the aid of the approximation means, such that said matrix of threads comes into contact with the surgical specimen;
(c) exposing the device to radiographic procedure, with said movable cover in the closed position;
(d) immersing the device in formaldehyde for an appropriate time, with said moveable cover in open position;
(e) moving said plates apart from each other, with the slackening of the approximation means, enabling the surgical specimen to be withdrawn, making it available for later evaluation.

### BRIEF DESCRIPTION OF THE DRAWINGS

The drawings mentioned below represent a particular embodiment of the invention, without imposing any limitation other than that contained in the claims.
- fig. 1A - top view of the upper plate (15) of a particular embodiment of the auxiliary device for locating, mapping and microscopically measuring neoplasias (10) of the invention;
- fig. 1B - top view of the cover (20) of the upper plate (15) shown in fig. 1A;
- fig. 1C - top view of the frame of the upper plate (15) shown in fig. 1A;
- fig. 2 - top view of the lower plate (45) of a particular embodiment of the invention;
- fig. 3 - side view of the set of bolt (50) and nut (55) joining the upper (15) and lower (45) plates of a particular embodiment of the device (10) of the invention;
- fig. 4 - perspective view of a particular embodiment of the device (10) of the invention, with the cover (20) of the upper plate (15) closed;
- fig. 5 - perspective view of a particular embodiment of the device (10) of the invention, with the cover (20) of the upper plate (15) open.
- fig. 6 - top view of the upper plate (15), in an alternative embodiment of the matrix of threads (35) situated beneath the cover (20).

### EXAMPLE

The following example, described with the aid of the accompanying drawings, is given as an illustration of a particular embodiment of the invention, without imposing restrictions thereon, other than those contained in the accompanying claims.

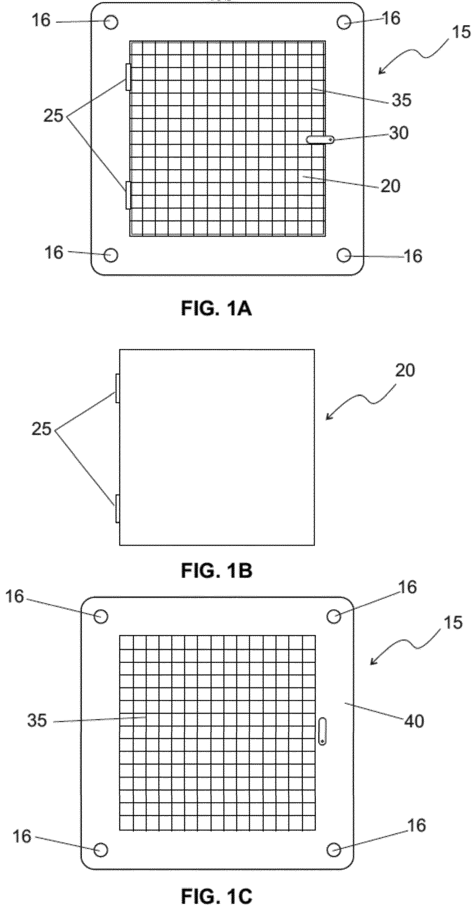

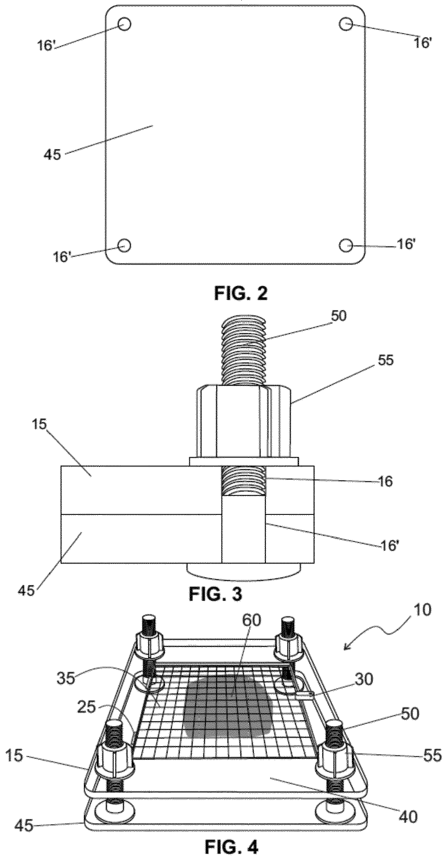

The device 10 of the invention comprises two cooperating plates, one upper plate 15 and one lower plate 45, with details presented in figures 1A, 1B, 1C and 2. The dimensions of the plates are 21cm x 21 cm (width x length).

The upper plate 15 comprises two cooperating parts, namely a frame 40 and a tilting central cover 20 (measuring 15cm x 15cm), said cover 20 cooperating with the frame 40 by way of hinges 25, and kept closed on a non-definitive basis by way of a lock 30.

The frame 40 and the central cover 20 of the upper plate 15 are made of radio-transparent acrylic, 10 mm and 5 mm in thickness, respectively.

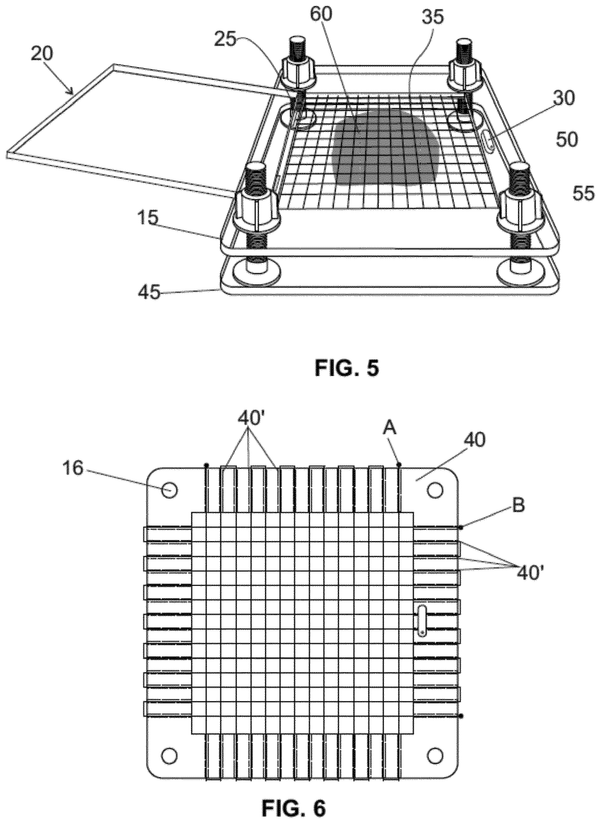

A matrix 35 of fine nylon threads measuring 0.6mm in diameter having low radio-opacity, is associated to the frame 40 of the upper plate, just beneath the central cover 20. In this example, the matrix is 14 strands by 14 strands, creating squares measuring 1cm x 1cm. In this example, as can be seen in fig. 6, the matrix is comprised of two strands A and B, which run perpendicular to each other, passing through the holes 40' of the frame 40, so as to form a checkered grid.

The lower plate 45 is solid, made of acrylic, having the same dimensions 21cm x 21 cm as the contour of the upper plate, and 10 mm thick.

As can be seen also with aid of fig. 3, the two plates are approachable to each other by means of 4 sets of bolts 50 and nuts 55 made of polyoxymethylene, each bolt 50 running through the plates (15 and 45) through holes 16 (of plate 15) and 16' (of plate 45) located at the corners of the plates (15 and 45). The picture is solely to illustrate the approximation means, as the plates 15 and 45 do not touch when the device 10 of the invention is in use.

Figures 5 and 6 show the device 10 containing a surgical specimen 60 kept between the plates 15 and 45, after tightening the nuts 55. Figure 5 shows the central cover 20 closed, and figure 6 shows the central cover 20 open.

As can be seen more clearly in fig. 5, the matrix of nylon threads 35 has direct contact with the surgical specimen 60, such that in the contact region, the squares are marked, which assists with the adequate subsequent location of the microcalcifications during the histological examination.

The central cover 20 remains closed until the device 10 is radiographed, and is opened when the device is immersed in formaldehyde.

It is known that a person skilled in the art, based on the information and exampled contained in this document, can make particular embodiments of the invention not specifically described, but which perform the same or similar functions to achieve results of the same nature. Such equivalent embodiments are encompassed by the accompanying claims.

## Claims

1. AUXILIARY DEVICE (10) FOR LOCATING, MAPPING AND MICROSCOPICALLY MEASURING NEOPLASIAS, **characterized by** comprising: - an upper plate (15) and a lower plate (45), having dimensions sufficient to contain a surgical specimen between them; - said upper plate (15) comprising a frame (40), a movable center cover (20) cooperating with said frame (40), and a matrix (35) of threads or strands; said matrix (35) situated beneath said central cover; - said upper plate (15) endowed with means (30) suitable to keep said movable center cover closed (20); - said matrix (35) being positioned so as to be in physical contact with the surgical specimen contained between the two said plates (15) and (45); - said movable cover (20) being made of material wholly or substantially radio-transparent; - said lower plate (15) being made of material substantially radio-transparent at least in the central region corresponding to the size of the movable cover (20) of the upper plate (15); - said upper plate (15) and lower plate (45) being cooperative with each other so as to be substantially parallel when the surgical specimen is immobilized, via approximation means (50, 55).

2. The DEVICE (10) as claimed in claim 1, characterized wherein said cover (20) is tilting in relation to said frame (40).

3. The DEVICE (10) as claimed in claim 1, characterized wherein the threads of the matrix (35) are made of nylon having reduced radio-opacity.

4. The DEVICE as claimed in claim 1, **characterized by** the fact that there are markings associated to said frame (40).

5. The DEVICE (10) as claimed in claim 1 characterized wherein said component parts are chemically resistant to contact with formaldehyde.

6. The DEVICE (10) as claimed in claim 1 characterized wherein the material wholly or substantially radio-transparent is methyl methacrylate.

7. The DEVICE (10) as claimed in claim 1 characterized wherein said approximation means between the upper (15) and lower (40) plates is one or more sets of bolts (50) and nuts (55).

8. The DEVICE (10) as claimed in claim 7 characterized wherein said bolt (50) and nut (55) are made of polyoxymethylene.

9. The DEVICE (10) as claimed in claim 1 **characterized by** being additionally endowed with slanting means.

10. USE OF THE AUXILIARY DEVICE (10) FOR LOCATING, MAPPING AND MICROSCOPICALLY MEASURING NEOPLASIAS as claimed in any of claims 1 to 9, **characterized by** comprising the following steps: (a) placing a surgical specimen (60) between said upper (15) and lower (45) plates; (b) bringing said plates (15 and 45) close to each other, pressing the surgical specimen between them (60) with the aid of the approximation means (50, 55), such that said matrix of threads (35) comes into contact with the surgical specimen (60); (b) exposing the device (10) to a radiographic procedure, with said movable cover (20) in the closed position with the aid of closing means (30); (c) immersing the device in formaldehyde for an appropriate time, with said movable cover (20) in open position, with the aid of moving means (25); (d) moving said upper (15) and lower (45) plates apart, by slacking the approximation means enabling the surgical specimen (60) to be withdrawn.
